# EUROPEAN PATENT APPLICATION

(11) **EP 4 640 215 A1**
(43) Date of publication of application: **29.10.2025**
(21) Application number: 23853694.0
(22) Date of filing: 22.12.2023
(51) Int. Cl.: A61K 9/51, A61K 31/502, A61K 31/75, A61K 45/06

(54) **NANOPARTICLES TO ENHANCE THE EFFECTS OF DRUGS FOR THE TREATMENT OF CANCER**

(30) Priority: 22.12.2022 ES 202231095
(71) Applicant: Universidad de Granada, 18071 Granada (ES)
(72) Inventor: PRADOS SALAZAR, José Carlos, 18071 Granada (ES); DELGADO LÓPEZ, José Manuel, 18071 Granada (ES); QUIÑONERO MUÑOZ, Francisco, 18071 Granada (ES); PARRA TORREJÓN, Belén, 18071 Granada (ES); RAMÍREZ RODRÍGUEZ, Gloria Belén, 18071 Granada (ES); MELGUIZO ALONSO, Consolación, 18071 Granada (ES); ORTIZ QUESADA, Raúl, 18071 Granada (ES); JIMÉNEZ LUNA, Cristina, 18071 Granada (ES)
(74) Representative: San Martin Alarcia, Esther
(86) International application number: PCT/ES2023/070781
(87) International publication number: WO 2024/134004

(57) **Abstract**

Biocompatible and biodegradable nanoparticles based on amorphous calcium phosphate containing ascorbic acid and anti-tumor agents. The use of these nanoparticles as highly stable nanocarriers of these drugs improves their effectiveness in terms of bioavailability, cytotoxicity, induction of apoptosis, inhibition of cell migration, tumor growth and survival.

## Description

### TECHNICAL FIELD

The present invention is framed in the field of medicine and, specifically, in the field of nanomedicine for the improvement of the treatment of tumor pathology. The invention focuses on the use of nanotechnology applied to the improvement of the drugs available for the treatment of cancer, reducing their toxic effects, with special attention to PARP inhibitor drugs.

### STATE OF THE ART

Pancreatic cancer (PC) is one of the most aggressive tumors and a leading cause of cancer-related death. Between 80-85% of patients are diagnosed at a too advanced stage, which precludes resection of the tumor. Even so, the survival rate of patients after resection is usually relatively low (around 20% at 5 years) and there are no really effective chemotherapeutic agents when the tumor is unresectable.

Among the available chemotherapeutic agents, Gemcitabine has been the agent of choice since the 1990s. Its results have improved with the co-treatment of Gemcitabine with nab-Paclitaxel, resulting in an increase in the patient's half-life. Other treatments follow this approach of combining drugs, such as FOLFIRINOX, which is a combination of 5-fluorouracil, leucovorin, irinotecan and oxaliplatin, and which offers better response rates. However, the survival rate is still very low and new and more effective treatments are needed to improve the quality of life of the patient.

One of the causes of the failure of therapy against this type of cancer is the development of resistance to the drugs with which it is treated, with poly ADP-ribose polymerase (PARP) being a relevant protein in the mechanism by which this resistance operates. Available PARP inhibitors (PARPi) include Olaparib (OLA), Rucaparib, Veliparib, Niraparib, Talazoparib and Pamiparib, although the most popular is the first one. In recent years, these PARP inhibitors have demonstrated efficacy in breast and ovarian cancer cells that possess depletion in DNA repair genes, especially BRCA (Faraoni & Graziani, 2018). In addition, it has been shown that PARP1 inhibition can reduce cell migration capacity in cancer cells (Mao et al., 2017; Prasad et al., 2017; Quiñonero et al., 2022), which is associated with tumor progression, invasiveness and metastasis generation. Its antitumor activity has also been studied in association with radiotherapy and chemotherapy in solid tumors. Indeed, the relevance of using PARPi as chemo-potentiators or chemo- and radiosensitisers in cancer and to overcome anti-cancer drug resistance has been demonstrated (Wang et al., 2022).

Specifically, Olaparib (OLA) has demonstrated a significant anti-tumor effect when used in combination with other cytotoxic agents such as 5-FU in colon cancer, Temozolomide in glioblastoma and Gemcitabine in pancreatic cancer (de Castro e Gloria et al., 2021; Hwang et al., 2021; Quiñonero et al., 2022; Waissi et al., 2021). Indeed, OLA was authorized by the FDA for the treatment of patients who did not respond to platinum-based chemotherapy and in tumors with the presence of BRCA mutations and has been studied as maintenance therapy in patients with BRCA-mutated metastatic tumors (Golan et al., 2019). However, its use in pancreatic cancer was accompanied by limiting side effects (Vaishampayan, 2021).

An important limitation of PARPi is related to its poor water solubility and its low bioavailability and retention in the tumor area. Therefore, optimizing the pharmacokinetic properties of PARPi and/or its associations is a crucial factor for its clinical use.

In order to overcome these limitations, nanotechnology has been suggested as a strategy to improve bioavailability, reduce systemic toxicity, improve targeting and increase the therapeutic outcome of the FDA-approved treatment regimen (Chowdhury et al., 2021). The ability to tailor the properties of nanoparticles (NPs) through shape, size (10-100 nm), active surface area and protection of charged species from degradation has opened the door to their use for drug delivery in cancer treatments. NPs overcome some drawbacks of chemotherapy, stay longer in the bloodstream, and accumulate in tumor sites thanks to the so-called EPR effect (enhanced permeability and retention), making treatment more effective (Kalyane et al., 2019; Byrne et al., 2008). In addition, nanoparticles are able to release drugs in a controlled manner in response to external stimuli such as pH, temperature or specific magnetic fields, thus improving the efficacy and non-toxicity of drugs. This way, nanotechnology has opened new horizons in the treatment of some aggressive and poorly-surviving cancers. In fact, OLA-containing NPs have already been synthesized and tested for the treatment of breast, small cell lung (Caster et al., 2015) and prostate cancer.

Despite the great development of chemotherapeutic nanocarriers in recent years, their application in pancreatic cancer has not been approved because the necessary studies related to nanotoxicity have not been carried out (Nedelcu et al., 2021).

In this scenario, calcium phosphate NPs, in the form of nanocrystalline apatite (Ap) or its precursor phase, amorphous calcium phosphate (ACP), have attracted scientific attention due to structural and chemical analogies with the inorganic nanostructure of bones and teeth (Lim et al. al., 2015) and their biocompatibility and biodegradability (Epple, 2018). In addition, its ability to accept other ions and its high specific surface area available to adsorb active biomolecules make this material an ideal nanoplatform to transport drugs or probes for teragnosis (Barbanente et al., 2020). This nanosystem is capable of releasing attached cargo to tumor sites in response to acidic pH conditions (Rodriguez-Ruiz et al., 2013), like the one found in the tumor microenvironment and cell lysosomes. In particular, calcium-containing nanosystems are of special interest, due to the imbalance in calcium homeostasis generated by their dissolution and their effect on cancer (Guan et al., 2020). Recently, Chen et al. (2022) demonstrated that the use of calcium phosphate spheres produced a synergistic effect between the encapsulated drug, in this case 5-FU, and the calcium (Ca²⁺ ) released by the NP, capable of inducing the production of reactive oxygen species (ROS).

In this line, ascorbic acid (AA) has been studied as a compound that could enhance the effect of PARP inhibitors when used in combinatorial therapy (Demiray, 2020) as a potential tumor cell-specific prooxidant agent that generates ROS such as hydrogen peroxide (H₂ O₂) and alters the redox metabolism of active labile iron in cancer cells, whereas in healthy cells the compounds are rapidly metabolized (Schoenfeld et al., 2017). Therefore, AA could be an appropriate and selective agent to damage tumor DNA, enhancing the efficacy of PARP inhibitors such as OLA (Demiray, 2020).

### DESCRIPTION OF THE INVENTION

The invention solves the problem of solubility of the agent Olaparib (OLA), allows its association with an effect enhancer such as ascorbic acid (AA) and increases the low activity of PARP inhibitor agents when used individually.

In the present invention, biocompatible nanoparticles (NPs) based on amorphous calcium phosphate (ACP) have been developed as nanocarriers for anti-tumor agents. Specifically, dual nanoplatforms containing Olaparib (OLA) and ascorbic acid (AA) are described for the treatment of pancreatic cancer (PC).

These nanoparticles are able to simultaneously release OLA and Ca²⁺, through slow and gradual dissolution of the NP, which enhances the antitumor effects associated with the drug. This effect is further enhanced by adding AA to the surface of the NP in order to improve the PARP inhibitory effect.

The OLA drug adsorbs on the surface of the calcium phosphate NPs of the invention with a loading efficiency of 75% and a loading of 13% (w/w). AA is also adsorbed on the surface of the NPs with a loading of 1% (w/w).

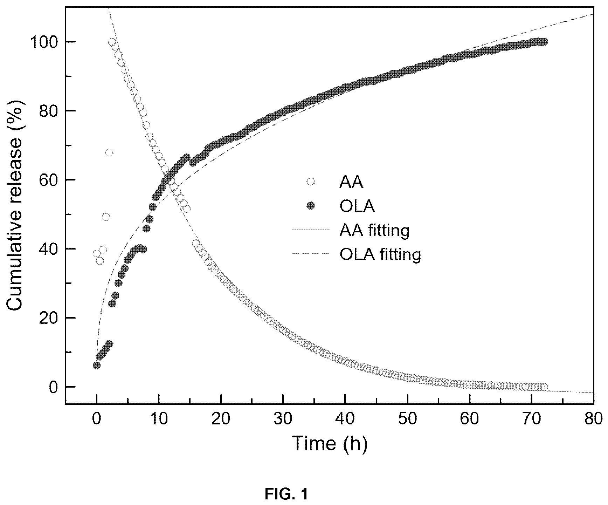

OLA is known to be very poorly soluble in water, but because it is bound to the NPs it is released slowly in the dissolution process **(****Fig. 1****),** which allows for a prolonged cellular uptake of OLA over time, improving the efficacy and efficiency of OLA treatment.

The effectiveness of NPs combining both molecules on the surface (NP-ACP-OLA-AA) is higher compared to free OLA (alone or combined with AA) in both *in vitro* and *in vivo* assays, where the antiproliferative effect and survival of mice is more favourable, evidencing the synergistic effect achieved with NPs.

*In vitro* studies were performed with three pancreatic cell lines (MIA PaCa-2, PANC1 and Panc02) showing that NPs containing OLA and AA (NP-ACP-OLA-AA) showed the best cytotoxic effect, outperforming free OLA.

These NP-ACP-OLA-AA showed greater genotoxicity (as measured by increased p-γ-H2AX expression) and a greater apoptosis-mediated cytotoxic effect than free OLA in PANC-1 cells. The combination of non-cytotoxic doses of AA together with OLA in PANC-1 cells generates a synergistic effect *in vitro* with greater toxicity than both compounds alone. This synergistic effect is related to the oxidation of AA that produces hydrogen peroxide that enters the tumor cells through peroxyporins, causing oxidative stress and DNA damage, and to the inhibition of the PARP1 enzyme (involved in DNA repair) caused by the PARP inhibitor in this case, OLA.

Adsorption of OLA on calcium phosphate NPs induced a similar cytotoxic effect on all cell lines tested and there were significant differences in the IC₅₀ achieved by the drugs.

These nanosystems were also more effective than AA-free nanoformulations despite having a very low surface AA payload (around 1%). Specifically, CI₅₀ values were 50, 56 and 28% lower for the Panc02, PANC-1 and MIA PaCa-2 lines, respectively, compared to free OLA. These results contradict the observations of Chakraborty & Jana (2017) who stated that AA increase oxidative stress only at high doses.

The results of the tests carried out demonstrate the high stability of NP-ACP-OLA-AA which delays its degradation in PBS medium for up to 60 h, and ensures the presence of OLA and AA during almost the entire *in vitro* treatment, and thus, longer synergistic effects between OLA and AA.

Furthermore, NP-ACP-OLA-AA enhanced the inhibition of the migration process in all tested cell lines compared to free OLA and significantly increased this inhibitory effect after 48 hours in PANC-1 cells compared to free OLA.

*In vivo* assays using NOD-SCID (Nonobese diabetic/severe combined immunodeficiency) mice with PANC-1-induced tumors revealed that NP-ACP-OLA-AA also reduced tumor volume to a greater extent (59.1%) than free OLA (28.3%) compared to untreated control mice. Increased intratumoral apoptosis was also observed with the use of these NPs compared to free OLA.

In addition, survival of mice after NP-ACP-OLA-AA treatment was increased (78%) compared to free OLA treatment (44%).

The results showed that calcium phosphate NPs, a highly stable, biocompatible and biodegradable system, are an ideal vector for co-treatment of OLA and AA in PC, inducing significant therapeutic benefits relative to free OLA, including cytotoxicity, induction of apoptosis, inhibition of cell migration, tumor growth and survival.

This therapy has been developed for pancreatic cancer, but can be extended to other tumors with certain types of drug resistance.

A first aspect of the invention relates to a nanoparticle, hereinafter "nanoparticle of the invention", based on amorphous calcium phosphate comprising ascorbic acid and at least one drug. Preferably, this is a drug for the treatment of cancer, and more preferably, the drug for the treatment of cancer is a PARP inhibitor. More preferably, the PARP inhibitor is selected from the list consisting of Olaparib, Rucaparib, Veliparib, Niraparib, Talazoparib and/or Pamiparib. In a more preferred embodiment it is Olaparib.

The nanoparticles of the invention are based on amorphous calcium phosphate (NP-ACP) which is synthesized from tribasic sodium citrate dihydrate (Na₃ (C₆H₅O₇)-2H₂O), anhydrous dibasic potassium phosphate (K₂HPO₄), sodium carbonate (Na₂CO₃) and calcium chloride dihydrate (CaCl₂ 2H₂ O).

The nanoparticles of the invention can present a percentage of the cancer treatment drug PARP inhibitor Olaparib, Rucaparib, Veliparib, Niraparib, Talazoparib and/or Pamiparib and more preferably Olaparib (OLA) of between 0.01% and 50% by weight of the total nanoparticle, even more preferably of between 5% and 25% and even more preferably of between 10% and 20%, the optimum percentage being around 12-15%. On the other hand, the ascorbic acid may be present in a percentage by weight over the total nanoparticle of between 0.001% and 10%, more preferably between 0.01% and 5%, and still more preferably between 0.1% and 3%, the optimum percentage being around 0.5- 2%.

The percentage adsorption efficiency of the cancer treatment drug PARP inhibitor Olaparib, Rucaparib, Veliparib, Niraparib, Talazoparib and/or Pamiparib and more preferably Olaparib (OLA), is preferably above 25%, more preferably above 50% and even more preferably is between 60 and 90%, the optimal percentage being around 65-85%.

A second aspect of the invention relates to the nanoparticle of the invention for its use as a medicament. Preferably, the medicament is used in the treatment of cancer. More preferably, the type of cancer to be treated is selected from the list consisting of breast cancer, small cell lung cancer, prostate cancer and/or pancreatic cancer. And in a more preferred embodiment, it is pancreatic cancer.

In a preferred embodiment of this aspect of the invention, the nanoparticle of the invention is used in a method of treating cancer in combination with at least one antitumor drug other than the one included in the nanoparticle. In a preferred embodiment, the anti-tumor drug with which it is combined is selected from the list consisting of 5-FU, Temozolomide and/or Gemcitabine.

Another aspect of the invention relates to a pharmaceutically acceptable composition, hereinafter composition of the invention, comprising the nanoparticle of the invention. In a preferred embodiment, the composition further comprises at least one antitumor drug other than the one included in the nanoparticle. In a more preferred embodiment the anti-tumor drug other than the one included in the nanoparticle is selected from the list consisting of 5-FU, Temozolomide and/or Gemcitabine.

Another aspect of the invention relates to composition of the invention for its use as a medicament. Preferably, the medicament is used in the treatment of cancer. More preferably, the type of cancer to be treated is selected from the list consisting of breast cancer, small cell lung cancer, prostate cancer and/or pancreatic cancer. And in a more preferred embodiment, it is pancreatic cancer.

"Pharmaceutically acceptable" is defined in this specification as a mixture of one or more active substances with or without additives, having physical characteristics for adequate dosage, storage, administration and bioavailability. "Active substance" is defined herein as a substance or combination of substances that has the capacity to exert pharmacological, immunological or metabolic action for the purpose of restoring, correcting or modifying physiological functions, or for diagnostic purposes. The term "drug" as used herein refers to any substance used for the prevention, diagnosis, alleviation, treatment or cure of diseases in humans and animals.

In another preferred embodiment of the present invention, the compositions and pharmaceutical forms of the invention are suitable for oral administration, in solid or liquid form. The compositions and pharmaceutical forms can be adapted for parenteral administration, as sterile solutions, suspensions, or lyophilisates of the products of the invention, using the appropriate dosage. Suitable excipients, such as pH buffering agents or surfactants, can be used.

Administration of the compounds, compositions or pharmaceutical forms of the present invention can be performed by any suitable method, such as intravenous infusion, oral, topical or parenteral routes. Oral administration is preferred for patient convenience.

The amount administered of the compound of the present invention will depend on the relative efficacy of the compound chosen, the severity of the disease to be treated and the weight, age and condition of the patient, the total dose, as well as the route of administration.

The compounds and compositions of the present invention can be employed together with other drugs in combination therapies. The other drugs may be part of the same composition or of a different composition, to be administered at the same time or at different times.

Another aspect of the invention relates to the method of obtaining the nanoparticle of the invention comprising the following steps:
a) synthesis of amorphous calcium phosphate nanoparticles by mixing a solution of CaCl₂ and Na₃C₆H₅O₇ and a solution of K₂HPO₄ and Na₂CO₃ ,
b) adsorption of the cancer drug, and
(c) adsorption of ascorbic acid.

Preferably, in step a) the mixture is stirred for 5 minutes.

Preferably in step b) the cancer treatment drug is a PARP inhibitor. Even more preferably the PARP inhibitor is selected from the list consisting of Olaparib, Rucaparib, Veliparib, Niraparib, Talazoparib and/or Pamiparib. In a more preferred embodiment it is Olaparib.

In another preferred embodiment, in step b) the amorphous calcium phosphate nanoparticles are added to a mixture of water with the cancer drug, PARP inhibitor, Olaparib, Rucaparib, Veliparib, Niraparib, Talazoparib and/or Pamiparib.

In a more preferred embodiment the cancer drug, PARP inhibitor, Olaparib, Rucaparib, Veliparib, Niraparib, Talazoparib and/or Pamiparib is pre-dissolved in DMSO. More preferably, the percentage of DMSO is less than 20% of the total volume of the mixture with water.

In an even more preferred embodiment, the drug is Olaparib and the mixture contains 1 gram of amorphous calcium phosphate nanoparticles and 132 mg of Olaparib, previously dissolved in DMSO at a concentration of 33.3 mg mL⁻¹, in 16 ml of water.

Preferably, in step b) an agitation of the mixture is carried out and more preferably, such agitation is carried out for 24 hours.

Preferably, in step b) a final centrifugation is carried out prior to step c) to collect the NP-ACP-OLA nanoparticles.

In step c) the NP-ACP-OLA nanoparticles are dispersed in ascorbic acid. More preferably, a 0.1 M ascorbic acid concentration is used and the pH is adjusted to 7. More preferably, 20.8 ml ascorbic acid is used per gram of NP-ACP-OLA nanoparticle.

Preferably, in step c) the mixture is stirred for 5 minutes.

Another aspect of the invention relates to nanoparticles obtained by the method of obtaining the invention.

Throughout the description and the claims the word "comprises" and its variants are not intended to exclude other technical features, additives, components or steps. To those skilled in the art, other objects, advantages and features of the invention will be apparent in part from the description and in part from the practice of the invention. The following examples and drawings are provided by way of illustration, and are not intended to be limiting of the present invention.

### DESCRIPTION OF THE FIGURES

**Figure 1****.** Release kinetics of OLA and AA from NPs. Cumulative release (%) of OLA and AA from NP-ACP-OLA-AA in PBS medium. AA degrades in solution once it separates from the surface of the NPs. The dashed line represents the theoretical curves estimated according to the power law approximation (*y(t)=a-t^{b}* ), where the amount of drug over time *(y(t))* depends on the effective diffusion constant *(a)*, time *(t)* and drug release mechanism *(b)*. The solid line represents the theoretical curves estimated according to an exponential decay of AA: *y(t)=exp(-t*/*τ), τ* corresponds to the half-life of AA.

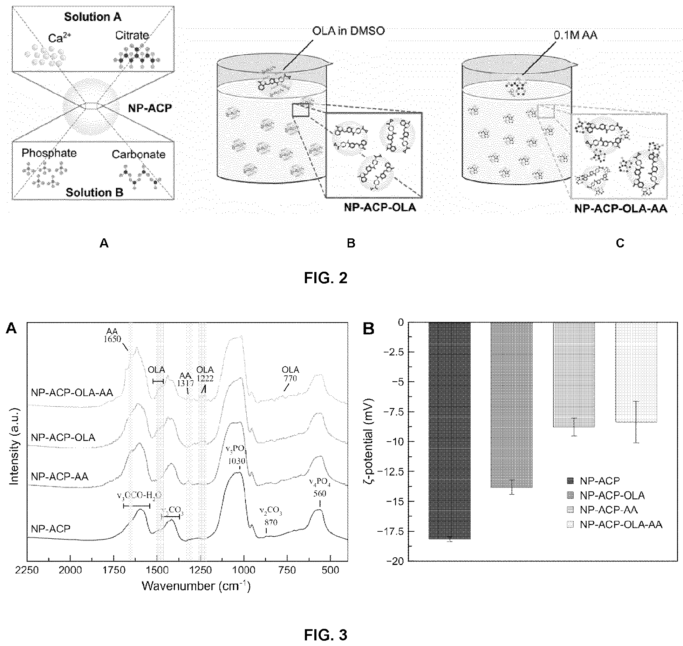

**Figure 2****.** Graphical scheme of NP functionalization to obtain the dual nanosystem. It consists of three steps: **(A)** synthesis of NP-ACP **(B)** adsorption of Olaparib (OLA) to create (NP-ACP-OLA) and **(C)** subsequent adsorption of ascorbic acid (NP-ACP-OLA-AA).
**Figure 3****.** Characterization of the nanosystems: **A)** FTIR spectra of control (NP-ACP) and ascorbic acid functionalized NPs (NP-ACP-AA), OLA (NP-ACP-OLA) and both molecules (NP-ACP-OLA-AA). The FTIR spectra were normalized by the maximum intensity (at approximately 1030 cm⁻¹) and shifted vertically for the sake of clarity. The bands attributed to OLA and ascorbic acid are highlighted in dark and light grey, respectively. **B)** Surface charge (ζ-potentials, mV) of functionalized (NP-ACP-AA, NP-ACP-OLA and NP-ACP-OLA-AA) and non-functionalized (NP-ACP) NPs. The graph shows the mean average and its standard deviation.

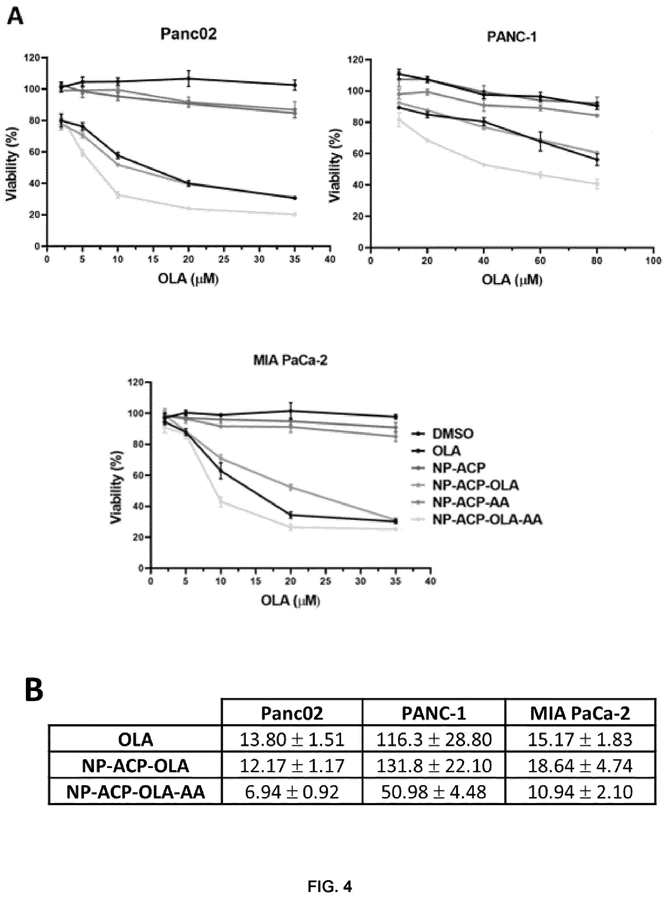

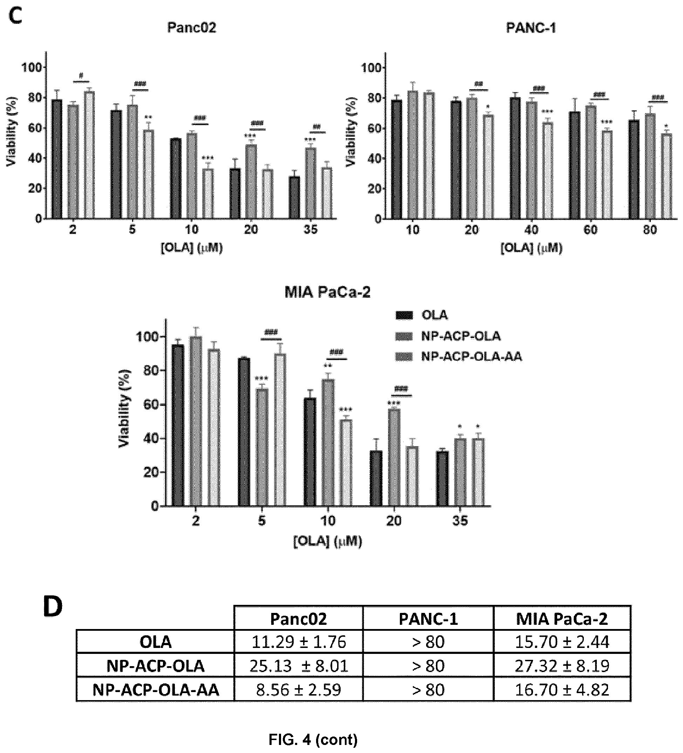

**Figure 4****.** Effect of free and NP-adsorbed Olaparib on different pancreatic adenocarcinomas and a colorectal cancer line. **(A)** Cell viability of pancreatic adenocarcinoma cell lines (Panc02, PANC-1 and MIA PaCa-2) exposed to increasing concentrations of OLA, free or NP-adsorbed. The cell viability of their controls (DMSO, NP-ACP and NP-ACP-AA) is also shown. **(B)** CI₅₀ values obtained in the cell lines after treatment with OLA, NP-ACP-OLA and NP-ACP-OLA-AA. **(C)** Relative proliferation values in cell lines treated with OLA or decorated NPs including OLA after subtraction of the effect of their controls. **(D)** CI₅₀ values obtained after calculation of relative proliferations. Data are represented as mean ± standard deviation (n=3). Asterisks indicated significant differences between the inhibition of free OLA against the same drug adsorbed at the same concentration in different nanoformulations * = p 0.05, ** = p 0.01, *** = p 0.001; # were used to indicate significant differences in cell viability between the NP-ACP-OLA and NP-ACP-OLA-AA groups. # = p 0.05, ## = p 0.01, ### = p 0.001.

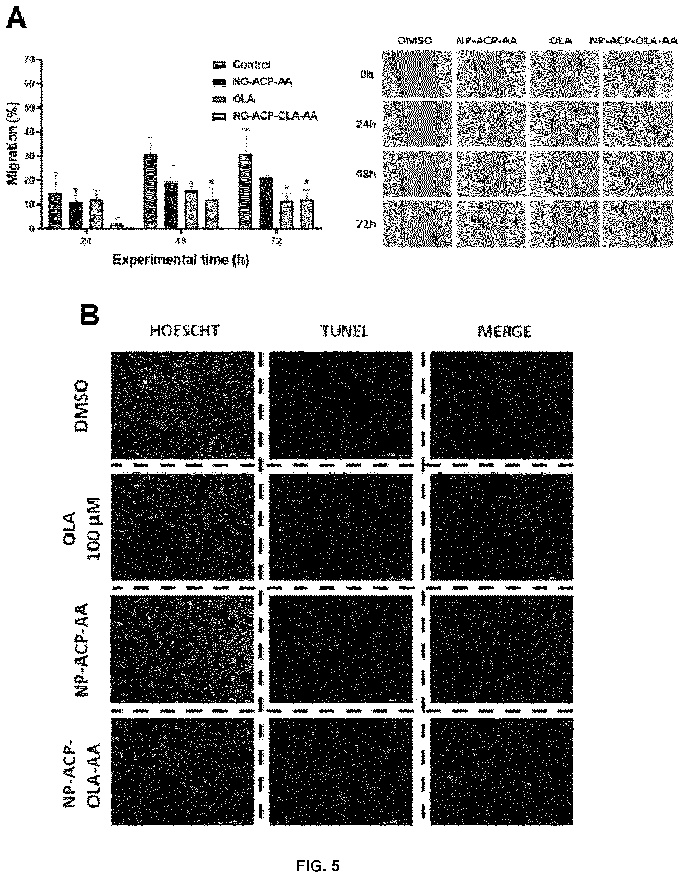

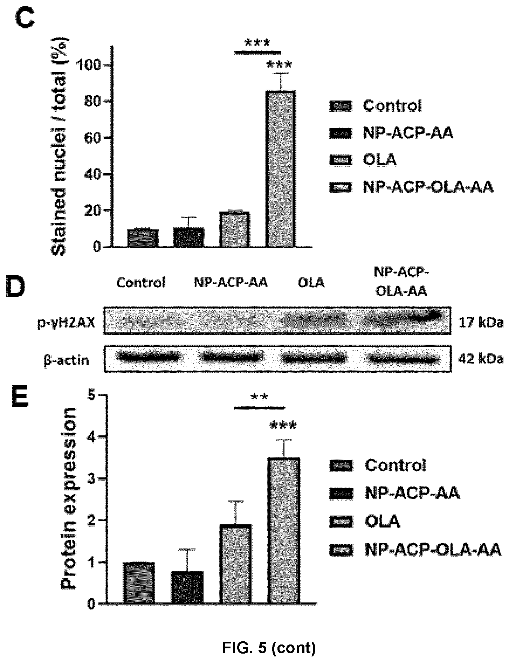

**Figure 5****.** **(A)** Results and representative images obtained after analysis of PANC-1 cell migration treated with NP-ACP-AA, free OLA and NP-ACP-OLA-AA for 72 h. **(B)** Representative fluorescence microscopy images obtained after performing a TUNEL assay on PANC-1 cells treated with NP-ACP-OLA-AA and free OLA. Nuclear labelling was performed by Hoechst staining. Pictures were taken with a 10X objective. **(C)** Quantification of TUNEL-stained nuclei compared to total nuclei. **(D)** Western blot of phosphorylated γ-H2AX after 24 h treatment of PANC-1 cells with OLA-free NPs, NP-ACP-AA and NP-ACP-OLA-AA. **(E)** Graph with protein expression results. All data are presented as mean ± standard deviation from three independent experiments. * = p ≤ 0.05, ** = p ≤ 0.01 and *** p ≤ 0.001.

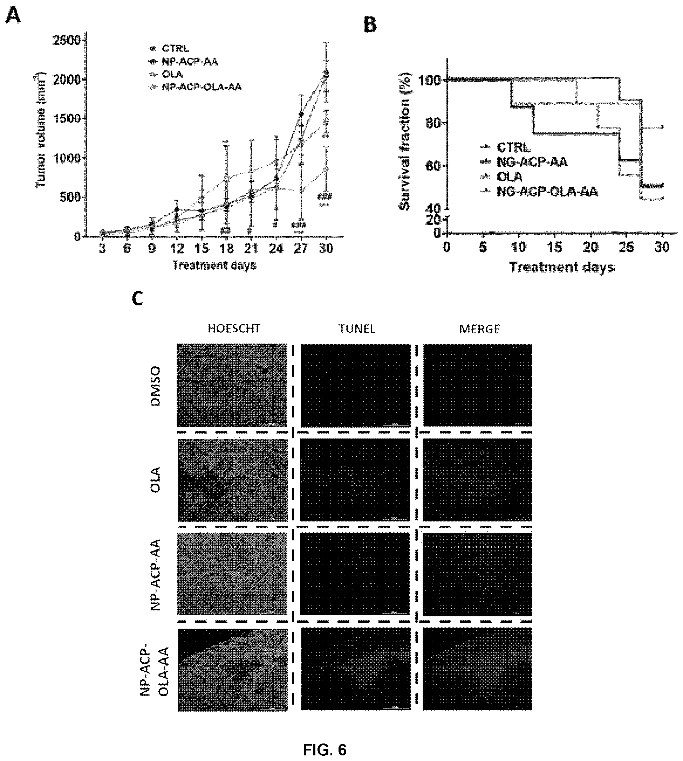

**Figure 6****.** *In vivo* effect of OLA-NP. Tumor growth **(A)** and survival of mice **(B)** after treatments with CTRL, OLA, NP-ACP-AA, OLA and NP-ACP-OLA-AA. **(C)** Histological assessment of apoptosis in resected tumors (TUNEL assay). Representative photographs of tumor sections derived from control (untreated) and NP-ACP-AA, OLA and NP-ACP-OLA-AA treated tumors showing TUNEL-positive cells. Sections were counterstained with Hoechst. Photographs were taken at 10X magnification. Asterisks indicate significant differences between the growth of all groups of mice versus the control group (CTRL). * = p ≤ 0.05, ** = p ≤ 0.01, *** = p ≤ 0.001; # was used to indicate significant differences between tumor growth in the free OLA-treated group (OLA group) versus the NP-ACP-OLA-AA-treated group of mice. # = p ≤ 0.05, ## = p ≤ 0.01, ### = p ≤ 0.001.

### DETAILED DESCRIPTION OF THE INVENTION

### Example 1: Optimization, synthesis, functionalization, characterization and drug release kinetics of nanoparticles

### Optimization of the synthesis and functionalization process

Tribasic sodium citrate dihydrate (Na₃(C₆H₅O₇)-2H₂ O, ≥99.0% pure), anhydrous dibasic potassium phosphate (K₂HPO₄ , ≥99.0% pure), sodium carbonate (Na₂CO₃ ≥99,0% pure), calcium chloride dihydrate (CaCl₂ 2H₂O ≥99.0% pure), ascorbic acid (C₆H₈O₆ ≥99.0% pure) and sodium hydroxide (NaOH ≥99.0 %, pellets for analysis) purchased from Sigma-Aldrich.

Olaparib (OLA) (C₂₄H₂₃FN₄O₃≥99 O.0 % pure powder) was purchased from MedChemExpress and anhydrous dimethyl sulfoxide (DMSO) ((CH₃) ₂ SO ≥99.0 %) was used to dissolve it. All solutions were prepared in ultrapure water (0.22 µS, 25 °C, Milli-Q, Millipore) and sterile conditions.

Amorphous calcium phosphate nanoparticles (ACP-NPs) were synthesized using a so-called biomimetic approach, in the presence of carbonate and citrate ions, with the aim of obtaining NPs with physicochemical properties similar to those of bone (Delgado-López et al., 2012). Briefly, two solutions (A) CaCl₂ (0.2 M) and Na₃Cit (0.2 M) and (B) K₂HPO₄ (0.12 M) and Na₂CO₃ (0.1 M) (1:1 v/v 200 ml in total) were mixed and shaken for 5 min. The NP-ACPs were collected and washed twice with ultrapure water by centrifugation (6000 rpm, 15 min, 10°C).

Dual functionalization of the NPs was carried out according to the steps illustrated in **Figure 2****.** First, calcium phosphate NPs were functionalized with OLA (NP-ACP-OLA), a molecule that is poorly soluble in water. In order to obtain the optimal conditions (maximum adsorption of OLA on the NPs), different ratios of NPs/OLA were mixed. The maximum loading was obtained by adding 1 g of NPs in 16 mL of water containing 132 mg of OLA (previously dissolved in DMSO at a concentration of 33.3 mg mL⁻¹). The mixture was stirred for 24h at room temperature to ensure maximum loading, in accordance with our previous studies aimed at functionalizing calcium phosphate NPs with doxorubicin, a poorly water-soluble chemotherapeutic agent (Rodriguez- Ruíz et al., 2013). Subsequently, the ACP-OLA NPs were collected by centrifugation (12500 rpm, 2 min, 10 °C). The optimal amount of water to disperse the nanoparticles was calculated from the volume of DMSO, which should not be higher than 20% of the total volume. In fact, it was observed that the OLA loading decreased considerably when the percentage of DMSO was higher than 20 % (e.g. 40 % v/v). During optimization, an experiment was also performed without using DMSO but it was observed that OLA did not adsorb on the NP, possibly due to its low solubility in water.

Then, NP-ACP-OLA was functionalized with ascorbic acid to obtain the dual NP (NP-ACP-OLA-AA). For this purpose, NP-ACP-OLA (1 g) was dispersed in 20.8 mL ascorbic acid (0.1 M, pH = 7 adjusted with NaOH). After 5 minutes of stirring at room temperature, NP-ACP-OLA-AA was collected by centrifugation (12500 rpm, 2 minutes, 10°C). Adsorption was performed for a short period of time (5 minutes) as AA is easily oxidized in aqueous solution and in the presence of oxygen. Also, we avoided performing the dual "one-pot" functionalization (initially adding the two molecules) or starting with ascorbic acid since OLA adsorption requires 24 h and in that time the complete oxidation of ascorbic acid takes place.

NP-ACP-AA were prepared as a control for biological assays by functionalizing ACP NPs with ascorbic acid (NP-ACP-AA), following the procedure described above. Samples containing AA were frozen at -80 °C to avoid degradation.

Small amounts of each sample were freeze-dried for further characterization and the rest of the sample was used for biological assays.

### Characterization and compositional analysis of doped NP-ACPs

Successful functionalization was assessed by FTIR spectroscopy and ζ-potential measurements.

Fourier transform infrared (FTIR) spectra of powder samples were recorded on a Tensor 27 spectrometer. Each sample (2 mg) was mixed with 200 mg of anhydrous potassium bromide (KBr), placed on a 12 mm diameter disc and pressed at 5 tonnes using a hydraulic press. Three discs were made for each sample and the KBr disc was the blank reference. All infrared spectra were recorded from 400 cm⁻¹ to 4000 cm⁻¹ at a resolution of 4 cm⁻¹. The nitrogen content of the NPs, NP-ACP-OLA and NP-ACP-OLA-AA, was measured by elemental analysis to quantify the amount of adsorbed OLA as well as the adsorption efficiency. This measurement was performed with a Thermo Scientific Flash 2000 elemental analyzer equipped with a microbalance. The amount of ascorbic acid adsorbed from NP-ACP-AA and NP-ACP-OLA-AA was quantified by UV-Vis spectroscopy at λ = 265 nm.

FTIR spectra **(****Figure 3A****)** of control (NP-ACP) and functionalized (NP-ACP-AA, NP-ACP-OLA and NP-ACP-OLA-AA) NPs show the typical poorly defined phosphate absorption bands characteristic of amorphous calcium phosphate with additional peaks corresponding to citrate, carbonate and water (Delgado-Lopez et al., 2012). The FTIR spectra of NP-ACP-AA and NP-ACP-OLA-AA **(****Figure 3A****)** also show two peaks at ~1317 cm⁻¹ (H-C-H bending) and 1650 cm⁻¹ (C=C ring stretching) attributed to ascorbic acid, confirming the successful adsorption of this molecule on the NP surface. The OLA signals (i.e. 1222 and 1438 cm-1) are distinguished in the FTIR spectra of NP-ACP-OLA and NP-ACP-OLA-AA, indicating that the adsorption of OLA was also effective.

Powder X-ray diffractograms (XRPD) were recorded on a Bruker AXS D8 Advance diffractometer using Cu Kα radiation (λ = 1.5418 Å), from 8° to 60° (2θ) with a scan rate of 0.5 s step⁻¹, step size of 0.02° with a 40 kV and 40 mA generator. Transmission electron microscopy (TEM) images, selected area electron diffraction (SAED), high-angle annular dark-field ring scanning transmission electron microscopy (HAADF-STEM) images and energy dispersive X-ray spectra (EDS) were acquired with STEM FEI TALOS Microscope F200X equipped with 4 Super-X SDDs (Thermo Fisher Scientific). The NP-ACP-OLA-AA were ultrasonically dispersed in ultrapure water and then drops of the suspension were deposited on 200 mesh copper grids coated with amorphous carbon thin films. The chemical composition of the NPs (Ca, P, K) was evaluated by ICP-OES (inductively coupled plasma optical emission spectroscopy, Optima 8300, PerkinElmer). 10 mg of the powdered samples were dissolved in 1 ml of ultrapure nitric acid and then the mixture was made up to 50 ml with ultrapure water. Each sample was measured in triplicate at the emission wavelength for the quantification of each element, Ca (317.93 nm) and P (213.62 nm). The surface charge of the NPs (zeta potential, ζ, mV) was measured on the Litesizer 500 (Anton Paar) by electrophoretic mobility and using disposable folded cells.

ζ-potential measurements were carried out to investigate the surface charge modification associated with NP functionalization **(****Figure 3B****).** The average ζ-potential of the non-functionalized ACP NPs was negative (-18 mV) due to citrate, which remains on the surface of the NPs. The ζ-potential increased to -14 mV after coupling with OLA. This increase was more pronounced for AA adsorption (NP-ACP-AA, **Figure 3B****),** with the reduction in surface charge being similar to that observed after dual functionalization (NP-ACP-OLA-AA). These findings confirmed the effective binding of AA and OLA on the surface. In fact, elemental analysis revealed the presence of 13.2 ± 0.9 wt.% OLA on NP-ACP-OLA-AA, achieving an adsorption efficiency of 75.4 ± 10.5 wt.%. On the other hand, the quantification of ascorbic acid by UV spectroscopy indicated a very low loading (1.17 ± 0.65 wt.%), most likely due to the few remaining active sites on the surface of the ACP NPs after OLA adsorption.

### Drug release kinetics of the active molecules in PBS media

The release kinetics of OLA and ascorbic acid from NP-ACP-OLA-AA was monitored by UV-Vis spectroscopy (Cary 100 Agilent Technologies). Briefly, 0.9 mg of NP was weighed into a quartz cuvette and then 3 mL of PBS solution (pH = 7.4) was carefully added to the cuvette. The absorbance curve from 200 nm to 400 nm was measured continuously every half hour until the plateau was reached. The amount of OLA and ascorbic acid released was determined at wavelengths of 311 nm and 265 nm, respectively. This technique allowed simultaneous quantification of the two molecules by monitoring the absorbance at 311 nm (OLA) and 265 nm (AA) **(****Figure 1****).** The experimental cumulative release of OLA was fitted to a semi-empirical power-law model, (*y(t)=a-t^{b}* , *b≤1*), where the parameter "a" refers to the release rate constant and "b" defines the drug release mechanism ("Mathematical models of drug release", 2015).

The dissolution kinetics of free OLA was also assessed by UV-Vis spectroscopy. For this, 3 mL of OLA solution (100 ppm) in PBS was placed in a quartz cuvette and the absorbance at 265 nm was measured continuously until the plateau was reached. The degradation rate of the ascorbic acid solution (10.5 ppm) in PBS was also monitored at 311 nm. All measurements were performed in triplicate.

**The desorption of OLA from the NPs had a slower release profile than the dissolution of free OLA, indicating the role of the NPs in slowing down the release/dissolution of the drug.**

The behaviour of AA is completely different. The concentration of AA in solution decreases with time **(****Figure 1****).** The degradation of AA followed an exponential decay according to the equation *y(t)=e*^{*-t*/*τ.*} , where τ corresponds to the half-life of the molecule. Interestingly, the half-life of AA released from NP-ACP-OLA-AA (τ = 15.3 h) is almost six times longer than the half-life of free AA in solution (τ = 2.7 h). In fact, free AA degraded within 12 h, while AA released from NP-ACP-OLA-AA remained for up to 60 h. This confirmed the protective effects of calcium phosphate NPs ensuring the availability of AA for almost the entire time of the *in vitro* cell experiments (72 h).

### Example 2: Cytotoxic effect of olaparib-containing nanoparticles on different pancreatic cancer cell lines

Human pancreatic ductal adenocarcinoma cell lines MIA PaCa-2 and PANC1 and the murine pancreatic cancer cell line Panc02 were used. All cell lines were cultured in Dulbecco's modified Eagle medium (DMEM) supplemented with 10% fetal bovine serum and 1% streptomycin penicillin (Sigma-Aldrich). Cells were maintained in monolayer culture in an atmosphere of 37°C and 5% CO₂ and passaged using 0.25% trypsin and 0.02% EDTA.

Panc02, PANC-1 and MIA PaCa-2 cell lines were treated with NP-ACP-OLA and NP-ACP-OLA-AA to determine their cytotoxic effect. The toxicity of NP-ACP and NP-ACP-AA (controls) was tested in all cell lines. For this, PANC-1 cells were seeded at densities of 8 × 10³ cells/well and Panc02 and MIA PaCa-2 cells at densities of 2.5 × 10³ cells/well in 48-well plates, incubated overnight and exposed to DMSO (OLA solvent), OLA (in different concentration ranges depending on the cell line used), NPs with and without OLA and ascorbic acid for 72h. Subsequently, the cells were fixed with 10% trichloroacetic acid for 20 minutes and washed three times with distilled water. Subsequently, the cells were stained with Sulforhodamine B (SRB) for 20 minutes, washed three times with 1% acetic acid. Subsequently, the SRB dye was solubilized using Trizma^{®} reagent. Finally, the optical density (OD) of the dye was measured with a Titertek Multiskan colorimeter (Flow) at 492 nm. Proliferation percentages (Pf%) were calculated as: Pf% = (OD of sample/OD of negative control) × 100. To calculate CI₅₀ values, a non-linear regression with viability curve fitting was performed using Prism 9.3. Compusyn 1.0 software was used to calculate the synergistic index of the combination of AA and OLA. NP-ACP at the highest concentration showed some inhibition of proliferation in the Pan02 cell line (20%). Furthermore, NP-ACP-OLA induced a cytotoxic effect similar to that of the OLA-free drug in all three cell lines tested **(****Fig. 4A****).** Interestingly, NP-ACP-OLA-AA enhanced the effect of the OLA-free drug by decreasing the CI₅₀ by 50, 56 and 28 % in Panc02, PANC-1 and MIA PaCa-2 lines, respectively **(****Fig. 4B****).**

Analysis of the toxicity of the nanoformulation relative to their respective controls showed a worse cytotoxic effect of NP-ACP-OLA than that of the OLA-free drug in all cell lines. Furthermore, the cytotoxic effect exerted by NP-ACP-OLA-AA on Panc02 and MIA PaCa-2 cells was similar to that of the OLA-free drug. In contrast, NP-ACP-OLA-AA induced a significantly greater cytotoxic effect than the OLA-free drug in the PANC-1 cell line, the most resistant cell line against OLA (CI₅₀ 7.5 and 8.5 times higher than in Panc02 and MIA PaCa-2, respectively), **(****Figs. 4C and 4D****).**

### Example 3: In vitro assays with NP-ACP-OLA-AA and the cell line PANC-1

### Cell migration assay

PANC-1 cell migration is a phenomenon related to PARP1 inhibition. To study the effect of NP-ACP-OLA-AA with respect to free OLA and OLA-NPs, the following treatment was carried out with the PANC-1 cell line. NP-ACP-AA was used as a control.

The PANC-1 cell line was seeded in a 12-well plate (Thermo Fisher) and grown to 95-100 % confluence in medium supplemented with DMEM (Sigma-Aldrich). Then, a vertical drop was manually performed using a pipette tip. The next day, dead cells were removed using phosphate buffered saline (PBS) and the culture medium was replaced with DMEM without FBS to prevent cell proliferation. Then, CI₂₅ of OLA concentration (free and OLA-NPs) was added to the wells and the cells were incubated for 72 h during which the migratory process was monitored using a DM-IL LED microscope. The percentage of migration was calculated by measuring the tumor cell free area at different times using ImageJ software with the Chemotaxis and Migration Tool add-on.

As shown in **Figure 5A****,** dual NP-ACP-OLA-AA nanoparticles significantly inhibited cell migration at 48 and 72 hours compared to untreated cells, while free OLA inhibited migration only at 72 hours, with no significant difference compared to the effect exerted with NPs **(****Fig. 5A****).**

### NP-ACP-OLA-AA-induced apoptosis: DNA fragmentation assay (TUNEL)

To determine the mechanism by which the NPs exerted their cytotoxic effect, a TUNEL assay was performed.

The TUNEL assay (Roche) was performed on PANC-1 cells and mouse PANC-1 tumor sections. Cells were seeded (1x 10⁵) in culture slide chambers (Franklin Lakes.), incubated overnight and treated with DMSO, NP-ACP-AA, OLA and NP-ACP-OLA-AA at 100 µM doses (24 hours). Cells treated with DMSO and NP-ACP-AA were used as control. Cells were then fixed in paraformaldehyde (4%) at room temperature for 1 hour and washed with PBS. Tissue sections were deparaffinised with xylol and ethanol mixtures of decreasing concentrations (100, 95, 70%) and rehydrated. Subsequently, both cells and tissues were permeabilised with Triton X100 (0.1 %) and sodium citrate solution diluted in PBS (0.1 %) for 2 and 8 min, respectively. After washing the samples with PBS, an experimental positive control was generated using 10 U/ml DNAase I (Sigma-Aldrich) for 10 min at room temperature. Finally, the TUNEL test was performed according to the manufacturer's instructions.

As shown in **Figure 5B****,** NP-ACP-OLA-AA induced a significantly greater pro-apoptotic effect in PANC-1 cells than the free drug. This effect was demonstrated by analyzing the percentage of TUNEL-positive nuclei, where NP-ACP-OLA-AA generated a higher induction of early apoptosis (86%) compared to the other conditions **(****Fig. 5C****).**

### NP-ACP-OLA-AA-induced genotoxicity: Western Blot transfer assays

The genotoxic capacity of these treatments was studied by the expression of phosphorylated γ-H2AX.

For this purpose, PANC-1 cells were washed twice with PBS and lysed with RIPA lysis buffer (Sigma-Aldrich). Protein concentration was determined using Bradford's reagent (Bio-Rad). For electrophoresis, 40 µg of proteins from each sample were heated at 95 °C for 5 min and separated on 12.5% SDS-PAGE gel. Fractions were transferred to nitrocellulose membranes, blocked for 1 h at room temperature in 5 % (w/v) BSA in TBS containing 0.1 % Tween 20 (TBS-T) and co-incubated overnight at 4 °C with the primary antibodies: γ-H2AX 1:1000 (Thermo Scientific) and β-actin-HRP 1:50000 (Santa Cruz Biotechnology). The membranes were then washed three times with TBS-T and incubated (1 h) with horseradish peroxidase (HRP)-conjugated anti-mouse secondary antibody 1:5000 (Santa Cruz Biotechnology). Proteins were visualized using the ECL system (Little Chalfont) on the LAS-4000 mini kit (GE Healthcare). Further analysis, as well as image processing and quantification of the bands, was performed using Quantity One analytical software (Bio-Rad). The expression of γ-H2AX was normalized to the β-actin level of the cell lines.

As shown in **Figures 5D and 5E****,** NP-ACP-OLA-AA generated a significant increase (86%) in genotoxicity in PANC-1 cells compared to free OLA after 72 hours of treatment.

### Example 4: In vivo assays with NP-ACP-OLA-AA and the cell line PANC-1

Having demonstrated the improved *in vitro* effect of OLA with the use of NPs in pancreatic adenocarcinoma lines, the *in vivo* effect was analyzed using SCID-NOD mice with PANC-1-induced tumors.

Male and female NOD scid gamma mice generated by the Centro de Instrumentación Cientifica of the University of Granada were used for the *in vivo* study. All mice (body weight: 20-25 g) were housed in cages with free access to water and food prior to the experiments and were maintained under controlled temperature, light and humidity (22.0 ± 2°C, 12 h light-dark cycle and 40-70% relative humidity), in specific pathogen-free conditions with sterile atmosphere controlled by HEPA filters. The study was approved by the Ethical Committee for Animal Experimentation of the University of Granada (reference code: 16/01/2020/005) and was performed in accordance with its guidelines.

Tumors were induced by subcutaneous injection of 9 x 10⁵ cells derived from the PANC1 cell line into the right flank of NOD-SCID gamma mice. When the tumor was palpable, animals were randomly divided into four groups (n= 8-10 mice per group, selecting the tumor-inducing mice): PANC1 mice treated with OLA solvent- (group I), OLA (group II), NP-ACP-AA (group III) and NP-ACP-OLA-AA (group IV). The OLA solvent was 5 % DMSO, 40 % PEG300, 5 % Tween-80 and 50 % saline and the NPs were dispersed in saline. All treatments were administered intraperitoneally. OLA was administered both free and encapsulated in NPs at a concentration of 25 mg/kg every 3 days for a total of 10 treatment cycles and the same volume of OLA solvent and concentration of free loading NPs were used in control and NP-ACP-AA control groups. Weights and deaths were carefully recorded throughout the period and tumor volume was measured with a digital caliper. Tumor volume (mm³) was calculated as follows: V (mm³ ) = (a × b2 × π) /6.

### Inhibition of tumor growth in vivo by Olaparib-decorated nanoparticles

As shown in **Figure 6A****,** the OLA solvent control (CTRL) and free loading NP-ACP-AA (control groups) showed similar final tumor volumes with no significant differences between them. In contrast, OLA treatment resulted in a decrease in tumor volume (28.3%) that was statistically significant in the last treatment cycle. Interestingly, the dual NP-ACP-OLA-AA nanosystem induced the largest decrease in tumor volume (59.1%), representing a significant decrease in tumor growth (30%) compared to free OLA.

In addition, as seen in **Figure 6B****,** differences in tumor growth were observed between the control groups in terms of survival (CTRL vs. NP-ACP-AA). In contrast, survival of mice after NP-ACP-OLA-AA treatment was higher (78%) compared to the control and free OLA treatment groups (50% and 44%, respectively) **(****Figure 6B****).** Finally, analysis of early apoptosis in histological sections obtained from mice indicated that OLA nanoformulations significantly increased the apoptotic potential of the drug **(****Figure 6C****).**

### Statistical analysis

The statistical tests used were performed with SPSS v.28.0 software. Thus, for the overall comparison between two samples, Student's t-tests were used. For the comparison between several samples, one-way ANOVA (for univariate comparisons) and two-way ANOVA (for comparisons of two variables) were performed with Tukey's post-hoc test, considering data with p < 0.05 as significant. A Kaplan-Meier method was used to determine the probability of mouse survival, and the log-rank test was used to compare the fraction of surviving mice between groups.

Therefore, the results of the *in vitro* activity of NP-ACP-OLA-AA were confirmed *in vivo.*

### Referencies

Barbanente, A., Nadar, R. A., Esposti, L. D., Palazzo, B., lafisco, M., Van Den Beucken, J. J. J. P., Leeuwenburgh, S. C. G., & Margiotta, N. (2020). Platinum-loaded, selenium-doped hydroxyapatite nanoparticles selectively reduce proliferation of prostate and breast cancer cells co-cultured in the presence of stem cells. Journal of Materials Chemistry B, 8(14), 2792-2804. https://doi.org/10.1039/d0tb00390e
Byrne, J. D., Betancourt, T., & Brannon-Peppas, L. (2008). Active targeting schemes for nanoparticle systems in cancer therapeutics. Advanced Drug Delivery Reviews, 60(15), 1615-1626. https://doi.org/10.1016/j.addr.2008.08.005
Caster, J. M., Sethi, M., Kowalczyk, S., Wang, E., Tian, X., Hyder, S. N., Wagner, K. T., Zhang, Y. A., Kapadia, C., Man Au, K., & Wang, A. Z. (2015). Nanoparticle delivery of chemosensitizers improve chemotherapy efficacy without incurring additional toxicity. Nanoscale, 7(6), 2805-2811. https://doi.org/10.1039/c4nr07102f
Chakraborty, A., & Jana, N. R. (2017). Vitamin C-Conjugated Nanoparticle Protects Cells from Oxidative Stress at Low Doses but Induces Oxidative Stress and Cell Death at High Doses. ACS Applied Materials and Interfaces, 9(48), 41807-41817. https://doi.org/10.1021/acsami.7b16055
Chen, J., Qiu, M., Zhang, S., Li, B., Li, D., Huang, X., Qian, Z., Zhao, J., Wang, Z., & Tang, D. (2022). A calcium phosphate drug carrier loading with 5-fluorouracil achieving a synergistic effect for pancreatic cancer therapy. Journal of Colloid and Interface Science, 605, 263-273. https://doi.org/10.1016/j.jcis.2021.07.080
Chowdhury, P., Ghosh, U., Samanta, K., Jaggi, M., Chauhan, S. C., & Yallapu, M. M. (2021). Bioactive nanotherapeutic trends to combat triple negative breast cancer. Bioactive Materials, 6(10), 3269-3287. https://doi.org/10.1016/j.bioactmat.2021.02.037
de Castro e Gloria, H., Jesuíno Nogueira, L., Bencke Grudzinski, P., da Costa Ghignatti, P. V., Guecheva, T. N., Motta Leguisamo, N., & Saffi, J. (2021). Olaparib-mediated enhancement of 5-fluorouracil cytotoxicity in mismatch repair deficient colorectal cancer cells. BMC Cancer, 21(1), 448. https://doi.org/10.1186/s12885-021-08188-7
Delgado-Lopez, J. M., lafisco, M., Rodriguez, I., Tampieri, A., Prat, M., & Gómez-Morales, J. (2012). Crystallization of bioinspired citrate-functionalized nanoapatite with tailored carbonate content. Acta Biomaterialia, 8(9), 3491-3499. https://doi.org/10.1016/j.actbio.2012.04.046
Demiray, M. (2020). Combinatorial Therapy of High Dose Vitamin C and PARP Inhibitors in DNA Repair Deficiency: A Series of 8 Patients. Integrative Cancer Therapies, 19, 1534735420969812. https://doi.org/10.1177/1534735420969812
Epple, M. (2018). Review of potential health risks associated with nanoscopic calcium phosphate. Acta Biomaterialia, 77, 1-14. https://doi.org/10.1016/j.actbio.2018.07.036
Faraoni, I., & Graziani, G. (2018). Role of BRCA mutations in cancer treatment with poly(ADP-ribose) polymerase (PARP) inhibitors. Cancers, 10(12), 487. https://doi.org/10.3390/cancers10120487
Gallarate, M., Carlotti, M. E., Trotta, M., & Bovo, S. (1999). On the stability of ascorbic acid in emulsified systems for topical and cosmetic use. International Journal of Pharmaceutics, 188(2), 233-241. https://doi.org/10.1016/S0378-5173(99)00228-8
Golan, T., Hammel, P., Reni, M., Van Cutsem, E., Macarulla, T., Hall, M. J., Park, J.-O., Hochhauser, D., Arnold, D., Oh, D.-Y., Reinacher-Schick, A., Tortora, G., Algül, H., O'Reilly, E. M., McGuinness, D., Cui, K. Y., Schlienger, K., Locker, G. Y., & Kindler, H. L. (2019). Maintenance Olaparib for Germline BRCA-Mutated Metastatic Pancreatic Cancer. The New England Journal of Medicine, 381(4), 317-327. https://doi.org/10.1056/NEJMOA1903387
Guan, Q., Zhou, L. Le, Lv, F. H., Li, W. Y., Li, Y. A., & Dong, Y. Bin. (2020). A Glycosylated Covalent Organic Framework Equipped with BODIPY and CaCO3 for Synergistic Tumor Therapy. Angewandte Chemie - International Edition, 59(41), 18042-18047. https://doi.org/10.1002/anie.202008055
Hwang, K., Lee, J. H., Kim, S. H., Go, K. O., Ji, S. Y., Han, J. H., & Kim, C. Y. (2021). The combination PARP inhibitor olaparib with temozolomide in an experimental glioblastoma model. In Vivo, 35(4), 2015-2023. https://doi.org/10.21873/INVIVO.12470
Kalyane, D., Raval, N., Maheshwari, R., Tambe, V., Kalia, K., & Tekade, R. K. (2019). Employment of enhanced permeability and retention effect (EPR): Nanoparticle-based precision tools for targeting of therapeutic and diagnostic agent in cancer. Materials Science and Engineering C, 98, 1252-1276. https://doi.org/10.1016/j.msec.2019.01.066
Lim, E. K., Kim, T., Paik, S., Haam, S., Huh, Y. M., & Lee, K. (2015). Nanomaterials for theranostics: Recent advances and future challenges. Chemical Reviews, 115(1), 327-394. https://doi.org/10.1021/cr300213b
Mao, X., Du, S., Yang, Z., Zhang, L., Peng, X., Jiang, N., & Zhou, H. (2017). Inhibitors of PARP-1 exert inhibitory effects on the biological characteristics of hepatocellular carcinoma cells in vitro. Molecular Medicine Reports, 16(1), 208-214. https://doi.org/10.3892/MMR.2017.6568
Mathematical models of drug release. (2015). In Strategies to Modify the Drug Release from Pharmaceutical Systems (pp. 63-86). Elsevier. https://doi.org/10.1016/B978-0-08-100092-2.00005-9
Nedelcu, A., Mocan, T., Grapa, C., & Mocan, L. (2021). Recent advances in nanoparticle-mediated diagnosis and the treatment of pancreatic cancer. International Journal of Molecular Sciences, 22(15), 8060. https://doi.org/10.3390/ijms22158060
Prasad, C. B., Prasad, S. B., Yadav, S. S., Pandey, L. K., Singh, S., Pradhan, S., & Narayan, G. (2017). Olaparib modulates DNA repair efficiency, sensitizes cervical cancer cells to cisplatin and exhibits anti-metastatic property. Scientific Reports, 7(1), 1-15. https://doi.org/10.1038/s41598-017-13232-3
Quiñonero, F., Mesas, C., Muioz-Gamez, J. A., Jiménez-Luna, C., Perazzoli, G., Prados, J., Melguizo, C., & Ortiz, R. (2022). PARP1 inhibition by Olaparib reduces the lethality of pancreatic cancer cells and increases their sensitivity to Gemcitabine. Biomedicine & Pharmacotherapy, 155, 113669. https://doi.org/10.1016/j.biopha.2022.113669
Rodriguez-Ruiz, I., Delgado-López, J. M., Duran-Olivencia, M. A., lafisco, M., Tampieri, A., Colangelo, D., Prat, M., & Gómez-Morales, J. (2013). PH-responsive delivery of doxorubicin from citrate-apatite nanocrystals with tailored carbonate content. Langmuir, 29(26), 8213-8221. https://doi.org/10.1 021/la4008334
Schoenfeld, J. D., Sibenaller, Z. A., Mapuskar, K. A., Wagner, B. A., Cramer-Morales, K. L., Furqan, M., Sandhu, S., Carlisle, T. L., Smith, M. C., Abu Hejleh, T., Berg, D. J., Zhang, J., Keech, J., Parekh, K. R., Bhatia, S., Monga, V., Bodeker, K. L., Ahmann, L., Vollstedt, S., ... Allen, B. G. (2017). O2·- and H2O2-Mediated Disruption of Fe Metabolism Causes the Differential Susceptibility of NSCLC and GBM Cancer Cells to Pharmacological Ascorbate. Cancer Cell, 31(4), 487-500. https://doi.org/10.1016/j.ccell.2017.02.018
Vaishampayan, U. N. (2021). An evaluation of olaparib for the treatment of pancreatic cancer. Expert Opinion on Pharmacotherapy, 22(4), 521-526. https://doi.org/10.1080/14656566.2020.1837113
Waissi, W., Amé, J. C., Mura, C., Noël, G., & Burckel, H. (2021). Gemcitabine-based chemoradiotherapy enhanced by a PARP inhibitor in pancreatic cancer cell lines. International Journal of Molecular Sciences, 22(13), 6824. https://doi.org/10.3390/ijms22136825
Wang, N., Yang, Y., Jin, D., Zhang, Z., Shen, K., Yang, J., Chen, H., Zhao, X., Yang, L., & Lu, H. (2022). PARP inhibitor resistance in breast and gynecological cancer: Resistance mechanisms and combination therapy strategies. Frontiers in Pharmacology, 13, 967633. https://doi.org/10.3389/fphar.2022.967633

## Claims

1. Nanoparticle based on amorphous calcium phosphate comprising ascorbic acid and at least one drug.

2. Nanoparticle according to the preceding claim **characterized in that** the drug is a drug for the treatment of cancer.

3. Nanoparticle according to the preceding claim **characterized in that** the cancer drug is a PARP inhibitor.

4. Nanoparticle according to the preceding claim **characterized in that** the PARP inhibitor drug is selected from the list consisting of Olaparib, Rucaparib, Veliparib, Niraparib, Talazoparib and/or Pamiparib.

5. Nanoparticle according to the preceding claim **characterized in that** the PARP inhibitor drug is Olaparib.

6. Nanoparticle according to any one of claims 1 to 5 for its use as a medicament.

7. Nanoparticle according to the preceding claim for its use as a medicament in the treatment of cancer.

8. Nanoparticle according to the preceding claim **characterized in that** the cancer type is selected from the list consisting of breast, small cell lung, prostate and/or pancreatic cancer.

9. Nanoparticle according to claim 5 for its use in the treatment of pancreatic cancer.

10. Nanoparticle according to any one of claims 1 to 5 for its use in a method for treating cancer in combination with at least one antitumor drug other than the one included in the nanoparticle.

11. Nanoparticle according to the preceding claim **characterized in that** the anti-tumor drug with which it is combined is selected from the list consisting of 5-FU, Temozolomide and/or Gemcitabine.

12. A pharmaceutically acceptable composition comprising the nanoparticle according to any one of claims 1 to 5.

13. A pharmaceutically acceptable composition according to the preceding claim, further comprising at least one anti-tumor drug other than the one included in the nanoparticle.

14. A pharmaceutically acceptable composition according to the preceding claim **characterized in that** the anti-tumor drug other than the one included in the nanoparticle is selected from the list consisting of 5-FU, Temozolomide and/or Gemcitabine.

15. A pharmaceutically acceptable composition according to the preceding claim for its use as a medicament.

16. A pharmaceutically acceptable composition according to the preceding claim for its use as a medicament for the treatment of cancer.

17. A pharmaceutically acceptable composition according to the preceding claim **characterized in that** the type of cancer is selected from the list consisting of breast cancer, small cell lung cancer, prostate cancer and/or pancreatic cancer.

18. A method for obtaining the nanoparticle according to any one of claims 1 to 5 comprising the following steps:
a) synthesis of amorphous calcium phosphate nanoparticles by mixing a solution of CaCl₂ and Na₃C₆H₅O₇ and a solution of K₂HPO₄ and Na₂CO₃,
b) adsorption of the cancer drug, and
(c) adsorption of ascorbic acid.
